# EUROPEAN PATENT APPLICATION

(11) **EP 4 218 602 A1**
(43) Date of publication of application: **02.08.2023**
(21) Application number: 21872112.4
(22) Date of filing: 01.09.2021
(51) Int. Cl.: A61B 8/14

(54) **ULTRASONIC SYSTEM, AND METHOD FOR CONTROLLING ULTRASONIC SYSTEM**

(30) Priority: 24.09.2020 JP 2020160122
(71) Applicant: FUJIFILM Corporation, Tokyo 106-8620 (JP)
(72) Inventor: KOSHINO Riko, Tokyo 106-8620 (JP)
(74) Representative: Hughes, Andrea Michelle
(86) International application number: PCT/JP2021/032082
(87) International publication number: WO 2022/064981

(57) **Abstract**

An ultrasound system (1) includes an ultrasound probe (2); an image generation unit (22) that generates two-dimensional ultrasound images of a plurality of frames in which a region of interest of a breast of a subject is imaged by performing transmission and reception of an ultrasound beam with respect to the subject using the ultrasound probe (2); a volume rendering image generation unit (26) that generates a volume rendering image including the region of interest on the basis of the two-dimensional ultrasound images of the plurality of frames; and a reference information linking unit (27) that links the volume rendering image to reference information regarding the region of interest.

## Description

### BACKGROUND OF THE INVENTION

### 1. Field of the Invention

The present invention relates to an ultrasound system, and a control method of the ultrasound system which are for examining a breast of a subject.

### 2. Description of the Related Art

In the related art, an examination of a lesion part in the breast of the subject is performed by using an ultrasound diagnostic apparatus. An examination using a radiological diagnostic apparatus is often performed in advance before the examination using such an ultrasound diagnostic apparatus and a user such as a doctor often performs a diagnosis of the lesion part in the breast of the subject by checking both a radiation image captured by the radiological diagnostic apparatus and an ultrasound image captured by the ultrasound diagnostic apparatus.

Thus, JP2014-14489A discloses an image display device designates a region of interest suspected to be a lesion part of a breast in a radiation image, specifies an ultrasound image in which a portion corresponding to the designated region of interest is imaged, and displays the ultrasound image so that the user can smoothly perform a diagnosis.

### SUMMARY OF THE INVENTION

In many cases, a lesion part in a breast of a subject normally has a three-dimensional shape, and it is preferable to understand the three-dimensional shape of the lesion part in order for a user such as a doctor to perform an accurate diagnosis. In the technique of JP2014-14489A, the user can perform a diagnosis of a region of interest in the breast of the subject while observing both the radiation image and the ultrasound image including the region of interest suspected to be the same lesion part, but in some cases, it is difficult to understand the three-dimensional shape of the region of interest in detail only by observing the radiation image and the ultrasound image which are two-dimensional images.

The present invention has been made in order to solve such a problem in the related art, and an object of the present invention is to provide an ultrasound system and a control method of the ultrasound system which can improve a diagnostic accuracy for the region of interest.

An ultrasound system according to an aspect of the present invention includes an ultrasound probe; an image generation unit that generates two-dimensional ultrasound images of a plurality of frames in which a region of interest of a breast of a subject is imaged by performing transmission and reception of an ultrasound beam with respect to the subject using the ultrasound probe; a volume rendering image generation unit that generates a volume rendering image including the region of interest on the basis of the two-dimensional ultrasound images of the plurality of frames; and a reference information linking unit that links the volume rendering image to reference information regarding the region of interest.

The reference information linking unit can store identification information of a radiation image in which the region of interest is imaged, as the reference information in a tag of the volume rendering image.

In this case, the radiation image includes two radiation images obtained by imaging the region of interest from two different directions, and the reference information linking unit can store identification information of each of the two radiation images, as the reference information in the tag of the volume rendering image.

The reference information linking unit can store identification information of another two-dimensional ultrasound image in which the region of interest is imaged, as the reference information in a tag of the volume rendering image.

In this case, the other two-dimensional ultrasound image includes two two-dimensional ultrasound images obtained by imaging two tomographic planes orthogonal to each other for the region of interest, and the reference information linking unit can store identification information of each of the two two-dimensional ultrasound images, as the reference information in the tag of the volume rendering image.

The ultrasound system can further include an ultrasound diagnostic apparatus; and a server connected to the ultrasound diagnostic apparatus, and in this case, the ultrasound diagnostic apparatus can include the ultrasound probe, the image generation unit, the volume rendering image generation unit, the reference information linking unit, and a communication unit that transmits the volume rendering image linked to the reference information, to the server.

The ultrasound system can further include a viewer connected to the server, in which the viewer can include an input device through which a user performs an input operation, and a monitor that displays a radiation image or the two-dimensional ultrasound image in which the region of interest is imaged, and in this case, in a case where the region of interest in the radiation image or the two-dimensional ultrasound image displayed on the monitor is designated by the user via the input device, the volume rendering image including the region of interest can be displayed on the monitor.

In a case where the region of interest in the radiation image or the two-dimensional ultrasound image displayed on the monitor is designated by the user via the input device, the radiation image or the two-dimensional ultrasound image and the volume rendering image in each of which the region of interest is imaged can be displayed on the monitor.

The volume rendering image generation unit can generate the volume rendering image using surface rendering.

The ultrasound system can further include an image memory that stores the volume rendering image.

A control method of an ultrasound system according to another aspect of the present invention includes generating two-dimensional ultrasound images of a plurality of frames in which a region of interest of a breast of a subject is imaged by performing transmission and reception of an ultrasound beam with respect to the subject using an ultrasound probe; generating a volume rendering image including the region of interest on the basis of the two-dimensional ultrasound images of the plurality of frames; and linking the volume rendering image to reference information regarding the region of interest.

According to the present invention, an ultrasound system includes an ultrasound probe; an image generation unit that generates two-dimensional ultrasound images of a plurality of frames in which a region of interest of a breast of a subject is imaged by performing transmission and reception of an ultrasound beam with respect to the subject using the ultrasound probe; a volume rendering image generation unit that generates a volume rendering image including the region of interest on the basis of the two-dimensional ultrasound images of the plurality of frames; and a reference information linking unit that links the volume rendering image to reference information regarding the region of interest. Therefore, the diagnostic accuracy for the region of interest can be improved.

### BRIEF DESCRIPTION OF THE DRAWINGS

Fig. 1 is a block diagram illustrating a configuration of an ultrasound system according to an embodiment of the present invention.
Fig. 2 is a block diagram illustrating a configuration of a transmission and reception circuit in an embodiment of the present invention.
Fig. 3 is a block diagram illustrating a configuration of an ultrasound image generation unit in an embodiment of the present invention.
Fig. 4 is a schematic diagram illustrating an example of a two-dimensional ultrasound image generated in an embodiment of the present invention.
Fig. 5 is a schematic diagram illustrating an example of a volume rendering image generated in an embodiment of the present invention.
Fig. 6 is a diagram illustrating a configuration of a server in an embodiment of the present invention.
Fig. 7 is a diagram illustrating a configuration of a viewer in an embodiment of the present invention.
Fig. 8 is a flowchart illustrating an operation of an ultrasound system according to an embodiment of the present invention.
Fig. 9 is a schematic diagram illustrating an example of a two-dimensional ultrasound image and a volume rendering image that are displayed on a viewer-side monitor in an embodiment of the present invention.
Fig. 10 is a schematic diagram illustrating an example of a two-dimensional ultrasound image, a volume rendering image, and a radiation image that are displayed on a viewer-side monitor in an embodiment of the present invention.
Fig. 11 is a schematic diagram illustrating an example of a radiation image and a volume rendering image that are displayed on a viewer-side monitor in an embodiment of the present invention.

### DESCRIPTION OF THE PREFERRED EMBODIMENTS

Hereinafter, embodiments of the invention will be described with reference to the accompanying drawings.

The description of configuration requirements described below is given on the basis of the representative embodiment of the present invention, but the present invention is not limited to such an embodiment.

In the present specification, a numerical range represented using "to" means a range including the numerical values before and after "to" as a lower limit value and an upper limit value.

In the present specification, the terms "same" and "identical" include an error range generally allowed in the technical field.

### Embodiment

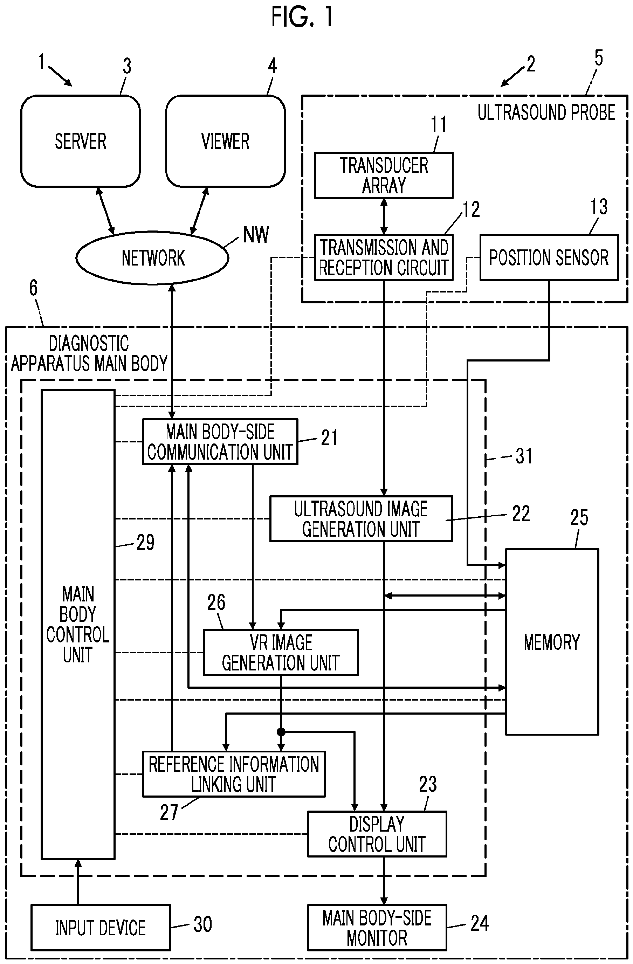

Fig. 1 illustrates a configuration of an ultrasound system 1 according to an embodiment of the present invention. The ultrasound system 1 includes an ultrasound diagnostic apparatus 2, a server 3, and a viewer 4. The ultrasound diagnostic apparatus 2 and the server 3 are connected to each other via a network NW. The server 3 and the viewer 4 are connected to each other via the network NW.

As illustrated in Fig. 1, the ultrasound diagnostic apparatus 2 includes an ultrasound probe 5 and a diagnostic apparatus main body 6. The ultrasound probe 5 includes a transducer array 11, and a transmission and reception circuit 12 is connected to the transducer array 11. A position sensor 13 is attached to the ultrasound probe 5.

The diagnostic apparatus main body 6 includes an ultrasound image generation unit 22, and the transmission and reception circuit 12 of the ultrasound probe 5 is connected to the ultrasound image generation unit 22. Further, a display control unit 23 and a main body-side monitor 24 are sequentially connected to the ultrasound image generation unit 22. A memory 25 is connected to the position sensor 13 of the ultrasound probe 5 and the ultrasound image generation unit 22. The diagnostic apparatus main body 6 includes a main body-side communication unit 21, and the main body-side communication unit 21 is connected to the server 3 via the network NW. The memory 25 is connected to the main body-side communication unit 21. A volume rendering (VR) image generation unit 26 is connected to the main body-side communication unit 21 and the memory 25. The display control unit 23 and a reference information linking unit 27 are connected to the VR image generation unit 26. The main body-side communication unit 21 and the memory 25 are connected to the reference information linking unit 27.

A main body control unit 29 is connected to the transmission and reception circuit 12, the position sensor 13, the main body-side communication unit 21, the ultrasound image generation unit 22, the display control unit 23, the memory 25, the VR image generation unit 26, and the reference information linking unit 27. An input device 30 is connected to the main body control unit 29.

The main body-side communication unit 21, the ultrasound image generation unit 22, the display control unit 23, the VR image generation unit 26, the reference information linking unit 27, and the main body control unit 29 constitute a main body-side processor 31.

The transducer array 11 of the ultrasound probe 5 illustrated in Fig. 1 has a plurality of ultrasonic transducers arranged in a one-dimensional or two-dimensional manner. According to a drive signal supplied from the transmission and reception circuit 12, each of the ultrasonic transducers transmits an ultrasonic wave and receives an ultrasound echo from a subject to output a signal based on the ultrasound echo. For example, each ultrasonic transducer is configured by forming electrodes at both ends of a piezoelectric body consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), or the like.

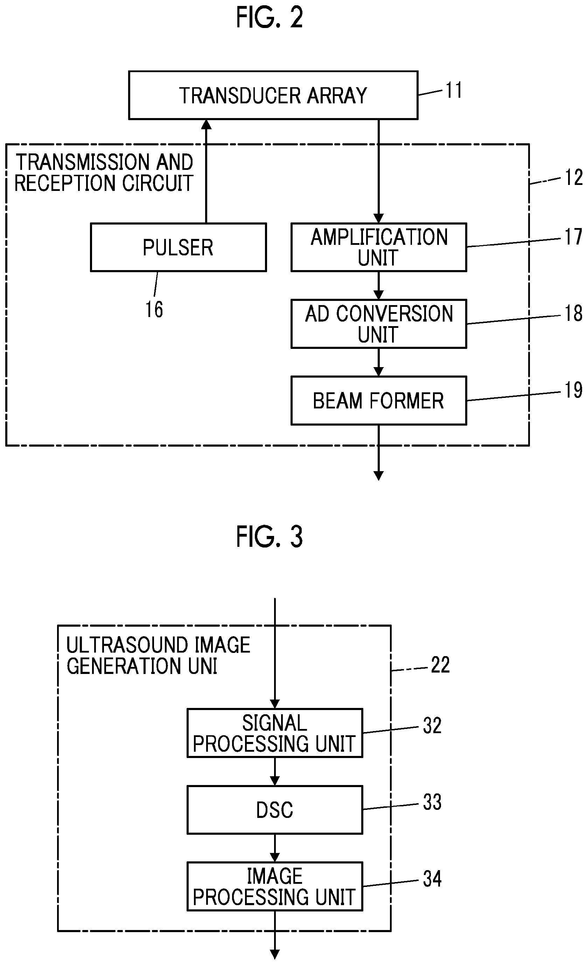

The transmission and reception circuit 12 causes the transducer array 11 to transmit the ultrasonic wave and generates a sound ray signal on the basis of a reception signal acquired by the transducer array 11, under the control of a probe control unit 15. As illustrated in Fig. 2, the transmission and reception circuit 12 has a pulser 16 connected to the transducer array 11, and an amplification unit 17, an analog digital (AD) conversion unit 18, and a beam former 19 that are sequentially connected in series from the transducer array 11.

The pulser 16 includes, for example, a plurality of pulse generators, and the pulser 16 adjusts the amount of delay of each drive signal so that ultrasonic waves transmitted from the plurality of ultrasonic transducers of the transducer array 11 form an ultrasound beam on the basis of a transmission delay pattern selected according to the control signal from the probe control unit 15, and supplies the obtained signals to the plurality of ultrasonic transducers. Thus, in a case where a pulsed or continuous-wave voltage is applied to the electrodes of the ultrasonic transducers of the transducer array 11, the piezoelectric body expands and contracts to generate pulsed or continuous-wave ultrasonic waves from each ultrasonic transducer. From the combined wave of these ultrasonic waves, an ultrasound beam is formed.

The transmitted ultrasound beam is reflected by a target, for example, a site of the subject, and propagates toward the transducer array 11 of the ultrasound probe 5. The ultrasound echo propagating toward the transducer array 11 in this manner is received by each ultrasonic transducer constituting the transducer array 11. In this case, each ultrasonic transducer constituting the transducer array 11 expands and contracts by receiving the propagating ultrasound echo to generate a reception signal that is an electric signal, and outputs the reception signal to the amplification unit 17.

The amplification unit 17 amplifies the reception signals input from each ultrasonic transducer constituting the transducer array 11, and transmits the amplified reception signals to the AD conversion unit 18. The AD conversion unit 18 converts the reception signal transmitted from the amplification unit 17 into digital reception data. The beam former 19 performs so-called reception focusing processing in which addition is performed by giving delays to respective pieces of the reception data received from the AD conversion unit 18. Through the reception focusing processing, a sound ray signal in which each piece of the reception data converted by the AD conversion unit 18 is phased and added and the focus of the ultrasound echo is narrowed is acquired.

The position sensor 13 is attached to the ultrasound probe 5, and detects positional information of the ultrasound probe 5. The positional information of the ultrasound probe 5 detected by the position sensor 13 is transmitted to the memory 25.

For example, as the position sensor 13, a magnetic sensor, an acceleration sensor, a gyro sensor, an optical position sensor, or a global positioning system (GPS) sensor can be used.

The main body-side communication unit 21 of the diagnostic apparatus main body 6 is configured by a circuit including an antenna for transmitting and receiving radio waves, and a circuit or the like for performing local area network (LAN) connection, and performs communication with the server 3 and the viewer 4 via the network NW under the control of the main body control unit 29. The main body-side communication unit 21 can receive identification information of the radiation image and the like from the server 3 via the network NW.

As illustrated in Fig. 3, the ultrasound image generation unit 22 has a configuration in which a signal processing unit 32, a digital scan converter (DSC) 33, and an image processing unit 34 are sequentially connected in series.

The signal processing unit 32 generates a B-mode image signal, which is tomographic image information regarding tissues inside the subject, by performing, on the sound ray signal received from the main body-side communication unit 21, correction of the attenuation due to the distance according to the depth of the reflection position of the ultrasonic wave using a sound speed value set by the main body control unit 29 and then performing envelope detection processing.

The DSC 33 converts (raster conversion) the B-mode image signal generated by the signal processing unit 32 into an image signal according to a normal television signal scanning method.

The image processing unit 34 performs various kinds of necessary image processing such as gradation processing on the B-mode image signal input from the DSC 33, and then sends the B-mode image signal to the display control unit 23 the memory 25. In the following, the B-mode image signal subjected to the image processing by the image processing unit 34 is simply referred to as a two-dimensional ultrasound image.

The memory 25 stores information such as the two-dimensional ultrasound image generated by the ultrasound image generation unit 22, the positional information of the ultrasound probe 5 acquired by the position sensor 13, and the radiation image transmitted from the server 3. In the memory 25, the positional information of the ultrasound probe 5 in a case where the two-dimensional ultrasound image is captured is stored in association with the two-dimensional ultrasound image each time the two-dimensional ultrasound image is stored in the memory 25, under the control of the main body control unit 29.

The two-dimensional ultrasound image and the positional information of the ultrasound probe 5 that are stored in the memory 25 are read out and sent to the VR image generation unit 26, under the control of the main body control unit 29.

The information on the radiation image or the like stored in the memory 25 is read out and sent to the reference information linking unit 27, under the control of the main body control unit 29.

For example, as the memory 25, recording media such as a flash memory, a hard disc drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), and a universal serial bus memory (USB memory), and the like can be used.

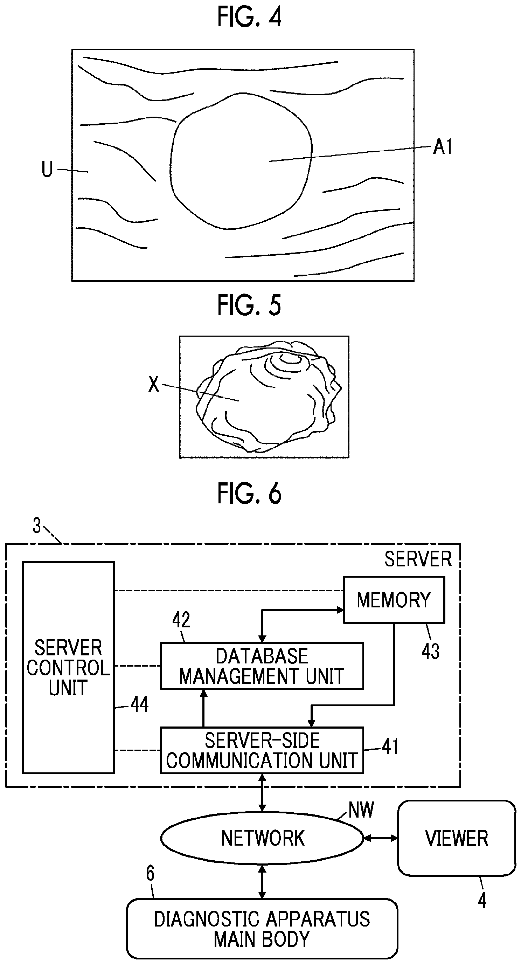

The VR image generation unit 26 performs an image analysis for each of two-dimensional ultrasound images of a plurality of frames stored in the memory 25, to automatically extract a region of interest A1 as illustrated in Fig. 4, and generates a volume rendering image X as illustrated in Fig. 5, which represents a three-dimensional shape of the region of interest A1, on the basis of the two-dimensional ultrasound images U of the plurality of frames and the positional information of the ultrasound probe 5 detected by the position sensor 13.

Here, the region of interest A1 is a region suspected to be a lesion part of the subject.

For example, the VR image generation unit 26 can store typical pattern data of the region of interest A1 in advance as a template, calculate a similarity degree for the pattern data while searching the two-dimensional ultrasound image, and consider that the region of interest A1 is present in a place where the similarity degree is equal to or greater than a threshold value and is the maximum.

As a method of extracting the region of interest A1, in addition to a method using simple template matching, for example, a machine learning method described in Csurka et al.: Visual Categorization with Bags of Keypoints, Proc. of ECCV Workshop on Statistical Learning in Computer Vision, pp. 59-74 (2004) or a general image recognition method using deep learning described in Krizhevsk et al.: ImageNet Classification with Deep Convolutional Neural Networks, Advances in Neural Information Processing Systems 25, pp. 1106-1114 (2012) can be used.

The VR image generation unit 26 can generate the volume rendering image X of the region of interest A1 by a so-called surface rendering method of extracting a contour of the region of interest A1 for the two-dimensional ultrasound images U of the plurality of frames and performing registration of the region of interest A1 using the information on the contour of the region of interest A1 extracted from the two-dimensional ultrasound images U of the plurality of frames, and the positional information of the ultrasound probe 5 in a case where each two-dimensional ultrasound image is captured.

The generation method of the volume rendering image X of the region of interest A1 is not limited to the surface rendering, and for example, a so-called volume rendering method may be used by using information on the entire region of interest A1 in the two-dimensional ultrasound images U of the plurality of frames.

The VR image generation unit 26 can generate the volume rendering image X, for example, by constructing a three-dimensional model of the region of interest A1 by a method such as surface rendering, displaying the three-dimensional model on the main body-side monitor 24, and taking a so-called screen shot of the three-dimensional model while the three-dimensional model is rotated, enlarged, and reduced on the main body-side monitor 24 by the user's input operation via the input device 30.

The reference information linking unit 27 links the volume rendering image X generated by the VR image generation unit 26 to reference information regarding the region of interest A1.

The reference information regarding the region of interest A1 is information including identification information on the radiation image in which the region of interest A1 is imaged, the identification information being transmitted from the server 3 to the main body-side communication unit 21 via the network NW, and identification information on the representative two-dimensional ultrasound image U among the two-dimensional ultrasound images U of the plurality of frames used in a case where the volume rendering image X is generated by the VR image generation unit 26.

Here, for example, by the VR image generation unit 26, the two-dimensional ultrasound image representing a tomogram passing through the center of the three-dimensional shape of the region of interest A1 can be selected on the basis of the positional information of the ultrasound probe 5 detected by the position sensor 13, and the identification information on the two-dimensional ultrasound image can be sent as the identification information on the representative two-dimensional ultrasound image U among the two-dimensional ultrasound images U of the plurality of frames used in a case where the volume rendering image X is generated, to the reference information linking unit 27.

The reference information linking unit 27 can generate a tag for storing information, for the volume rendering image X, and link the volume rendering image X to the reference information by storing the reference information including the identification information on the radiation image and the identification information on the two-dimensional ultrasound image U in the tag of the volume rendering image X.

Here, as the tag of the volume rendering image X, for example, so-called Digital Imaging and Communications in Medicine (DICOM) can be used. Specifically, as the reference information which is included in a DICOM tag of the volume rendering image X, and includes the identification information of the radiation image and the identification information of the two-dimensional ultrasound image U, Service Objective Pair Instance Unique IDentifiers (SOP Instance UID) of the images can be used. Further, as the reference information described above, in addition to the SOP Instance UID, a Study Instance Unique IDentifier (Study Instance UID), or a Series Instance Unique IDentifier (Series Instance UID) may be used.

The volume rendering image X linked to the reference information by the reference information linking unit 27 in this manner is transmitted from the main body-side communication unit 21 to the server 3 via the network NW.

The main body control unit 29 controls each unit of the ultrasound probe 5 and each unit of the diagnostic apparatus main body 6 according to a program and the like recorded in advance.

The display control unit 23 of the diagnostic apparatus main body 6 performs predetermined processing on the two-dimensional ultrasound image U generated by the ultrasound image generation unit 22 and the volume rendering image X generated by the VR image generation unit 26 to display the processed images on the main body-side monitor 24, under the control of the main body control unit 29.

The main body-side monitor 24 performs various kinds of display under the control of the display control unit 23. The main body-side monitor 24 includes a display device such as a liquid crystal display (LCD), or an organic electroluminescence (EL) display.

The input device 30 of the diagnostic apparatus main body 6 is for the user to perform an input operation. The input device 30 is configured by, for example, a device for a user to perform an input operation, such as a keyboard, a mouse, a trackball, a touchpad, a touch panel, or the like.

The main body-side processor 31 including the main body-side communication unit 21, the ultrasound image generation unit 22, the display control unit 23, the VR image generation unit 26, the reference information linking unit 27, and the main body control unit 29 is configured by a central processing unit (CPU) and a control program for causing the CPU to execute various kinds of processing, but the main body-side processor 31 may be configured by using a field programmable gate array (FPGA), a digital signal processor (DSP), an application specific integrated circuit (ASIC), a graphics processing unit (GPU), and another integrated circuit (IC) or may be configured by a combination thereof.

In addition, the main body-side communication unit 21, the ultrasound image generation unit 22, the display control unit 23, the VR image generation unit 26, the reference information linking unit 27, and the main body control unit 29 can also be configured by being integrated partially or entirely into one CPU or the like.

As illustrated in Fig. 6, the server 3 includes a server-side communication unit 41. A database management unit 42 is connected to the server-side communication unit 41. A memory 43 is connected to the database management unit 42, and the server-side communication unit 41 is connected to the memory 43. Further, a server control unit 44 is connected to the server-side communication unit 41, the database management unit 42, and the memory 43.

The server-side communication unit 41, the database management unit 42, and the server control unit 44 constitute a server processor (not illustrated).

The server 3 is installed in, for example, a hospital, and is installed at a location remote from a place where the diagnostic apparatus main body 6 is disposed. The server 3 is for managing image data, and is used in a case where the user such as a doctor performs a diagnosis for the region of interest A1 of the subject, for example. As a specific example, as the server 3, a so-called picture archiving and communication system (PACS) can be used.

Similar to the main body-side communication unit 21, the server-side communication unit 41 is configured by a circuit including an antenna for transmitting and receiving radio waves, and a circuit or the like for performing LAN, and performs communication with the viewer 4 and the diagnostic apparatus main body 6 via the network NW under the control of the server control unit 44.

The database management unit 42 associates the volume rendering image X with the image data on the two-dimensional ultrasound image U and the radiation image linked to the volume rendering image X on the basis of the information of the tag of the volume rendering image X transmitted from the diagnostic apparatus main body 6 to the server-side communication unit 41 via the network NW. In this manner, the database management unit 42 creates a database from the information regarding the volume rendering image X, and stores the databased information in the memory 43.

The memory 43 stores the volume rendering image X, the two-dimensional ultrasound image U, the radiation image, and other information of which the relationship is databased by the database management unit 42. The information stored in the memory 43 is sent to the server-side communication unit 41, and is transmitted to the viewer 4 and the diagnostic apparatus main body 6 via the network NW, under the control of the server control unit 44.

As the memory 43, for example, recording media such as a flash memory, an HDD, an SSD, an FD, an MO disc, an MT, a RAM, a CD, a DVD, an SD card, and an USB memory can be used.

The server control unit 44 controls each unit of the server 3 according to a program and the like stored in advance.

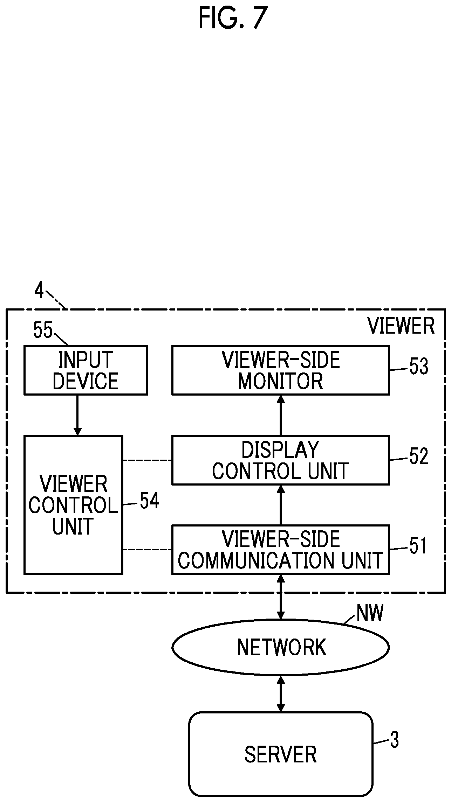

As illustrated in Fig. 7, the viewer 4 includes a viewer-side communication unit 51, and a display control unit 52 and a viewer-side monitor 53 are sequentially connected to the viewer-side communication unit 51. A viewer control unit 54 is connected to the viewer-side communication unit 51 and the display control unit 52. An input device 55 is connected to the viewer control unit 54.

The viewer 4 is installed in, for example, a hospital, and is installed at a location remote from the installation place of the diagnostic apparatus main body 6. For example, the viewer 4 is configured by a personal computer or the like, and is used in a case where the user such as a doctor browses the data stored in the server 3. As a specific example, as the viewer 4, a so-called PACS can be used.

Similar to the main body-side communication unit 21 and the server-side communication unit 41, the viewer-side communication unit 51 is configured by a circuit including an antenna for transmitting and receiving radio waves, and a circuit or the like for performing LAN, and performs communication with the server 3 via the network NW under the control of the viewer control unit 54.

The display control unit 52 of the viewer 4 performs predetermined processing on the two-dimensional ultrasound image U, the volume rendering image X, and the radiation image that are received from the server 3, and displays the processed images on the viewer-side monitor 53, under the control of the viewer control unit 54.

The viewer-side monitor 53 performs various kinds of display under the control of the display control unit 52. For example, the viewer-side monitor 53 includes a display device such as an LCD or an organic EL display.

The input device 55 of the viewer 4 is for the user to perform an input operation. The input device 55 is configured by, for example, a keyboard, a mouse, a trackball, a touchpad, a touch panel, or the like.

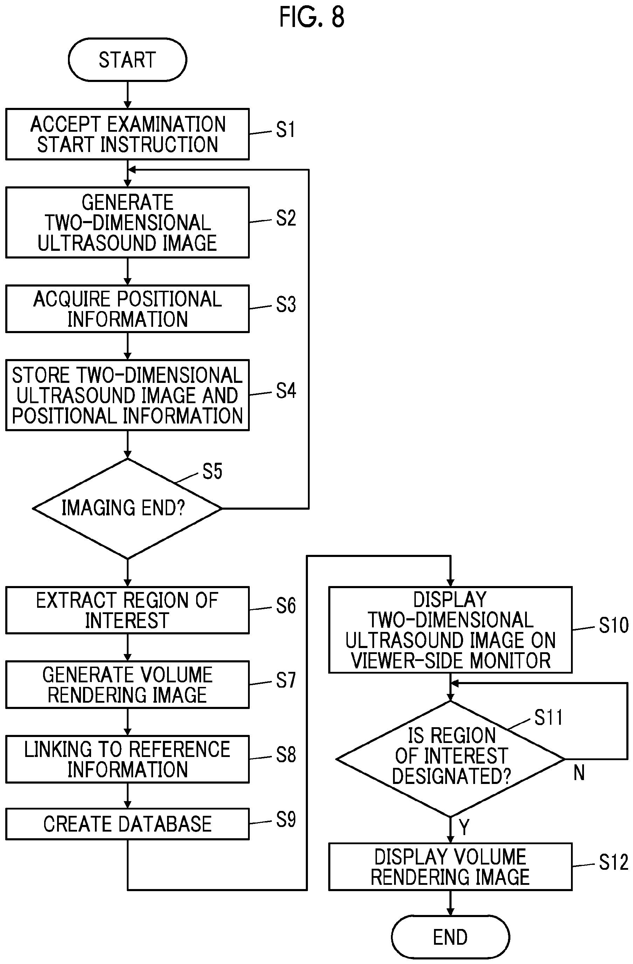

In the following, the operation of the ultrasound system 1 according to the embodiment of the present invention will be described using the flowchart illustrated in Fig. 8. In the description regarding the operation, an example will be described in which the radiation image in which the region of interest suspected to be a lesion part in the breast of the subject is imaged is stored in the memory 43 of the server 3, and the region of interest A1 that is the same as the region of interest included in the radiation image is examined using the ultrasound system 1 constructed in a hospital, for example.

First, in Step S1, the main body control unit 29 accepts an instruction to start an examination for the subject, which is input by the user's input operation via the input device 30 of the diagnostic apparatus main body 6. Thereby, the examination for the subject is started. In this case, a command to transmit the radiation image stored in the memory 43 of the server 3 to the diagnostic apparatus main body 6 is transmitted from the main body control unit 29 to the server 3 via the main body-side communication unit 21, and the radiation image is transmitted from the server 3 to the main body-side communication unit 21 of the diagnostic apparatus main body 6. The transmitted radiation image is sent from the main body-side communication unit 21 to the memory 25, and is stored in the memory 25.

Next, in Step S2, the two-dimensional ultrasound image U is captured while the ultrasound probe 5 is moved in a state of being in contact with the body surface of the subject by the user.

In this case, the transmission and reception circuit 12 performs reception focusing processing using a sound speed value set in advance to generate a sound ray signal under the control of the probe control unit 15. The sound ray signal generated by the transmission and reception circuit 12 in this manner is transmitted to the main body-side communication unit 21 of the diagnostic apparatus main body 6, and further sent to the ultrasound image generation unit 22. The ultrasound image generation unit 22 generates the two-dimensional ultrasound image U as illustrated in Fig. 4 using the sound ray signal received from the main body-side communication unit 21.

In subsequent Step S3, the positional information of the ultrasound probe 5 in a case where the two-dimensional ultrasound image U is generated in Step S2 is acquired by the position sensor 13 attached to the ultrasound probe 5.

In Step S4, the main body control unit 29 stores the two-dimensional ultrasound image U generated in Step S2 and the positional information of the ultrasound probe 5 acquired in Step S3 in the memory 25 of the diagnostic apparatus main body 6 in association with each other.

In Step S5, it is determined whether to end the capturing of the two-dimensional ultrasound image U. For example, in a case where an instruction to end the capturing of the two-dimensional ultrasound image U is input by the user's input operation via the input device 30 of the diagnostic apparatus main body 6, it is determined that the capturing of the two-dimensional ultrasound image U is to be ended, and in a case where an instruction to end the capturing of the two-dimensional ultrasound image U is not input, it is determined that the capturing of the two-dimensional ultrasound image U is continued.

In a case where it is determined that the capturing of the two-dimensional ultrasound image U is continued, the processing returns to Step S2, and the two-dimensional ultrasound image U is newly generated. Then, the positional information of the ultrasound probe 5 is acquired in Step S3, the two-dimensional ultrasound image U and the positional information of the ultrasound probe 5 are stored in the memory 25 in Step S4, and the processing proceeds to Step S5. In this manner, processing of Step S2 to Step S5 is repeated as long as it is determined that the capturing of the two-dimensional ultrasound image U is continued.

In a case where it is determined in Step S5 that the capturing of the two-dimensional ultrasound image U is to be ended, the processing proceeds to Step S6.

In Step S6, the VR image generation unit 26 automatically extracts the region of interest A1 suspected to be a lesion part from each of the two-dimensional ultrasound images U of the plurality of frames that are stored in the memory 25 by repeating Step S2 to Step S5.

Next, in Step S7, the VR image generation unit 26 generates the volume rendering image X as illustrated in Fig. 5 by performing registration of the region of interest A1 extracted from the two-dimensional ultrasound images U of the plurality of frames in Step S6, using the positional information of the ultrasound probe 5 that is stored in Step S4 in association with the two-dimensional ultrasound image U each time the two-dimensional ultrasound image U is generated. In this case, for example, the VR image generation unit 26 can extract the contour of the region of interest A1 in the two-dimensional ultrasound images U of the plurality of frames, and generate the volume rendering image X by using the surface rendering method.

In this manner, since the volume rendering image X of the region of interest A1 is generated, it is possible for the user to easily understand the three-dimensional shape of the region of interest A1 of the subject by checking the shape of the volume rendering image X.

In subsequent Step S8, the reference information linking unit 27 links the volume rendering image X generated in Step S7 to the reference information regarding the region of interest A1. In this case, for example, the reference information linking unit 27 generates a tag for storing information, for the volume rendering image X, and stores the identification information of the two-dimensional ultrasound image U of one representative frame among the two-dimensional ultrasound images U of the plurality of frames used in a case of generating the volume rendering image X in Step S7, as the reference information in the tag of the volume rendering image X. For example, the two-dimensional ultrasound image U of one representative frame is selected by the VR image generation unit 26, and is sent to the reference information linking unit 27.

Further, the VR image generation unit 26 stores the identification information of the radiation image stored in the memory 25 in Step S1, as the reference information in the tag of the volume rendering image X.

The volume rendering image X linked to the reference information is transmitted from the main body-side communication unit 21 to the server-side communication unit 41 of the server 3 via the network NW.

In this manner, since the volume rendering image X is not generated in the server 3, but is generated in the diagnostic apparatus main body 6, it is sufficient to transmit one volume rendering image X generated in the diagnostic apparatus main body 6 to the server 3, and for example, there is no need to transmit the two-dimensional ultrasound images U of the plurality of frames to the server 3. Therefore, for example, as compared with a case where the volume rendering image X is generated after the two-dimensional ultrasound images U of the plurality of frames are transmitted to the server 3, the server 3 can acquire the volume rendering image X more quickly, and the user can use the server 3 to perform a diagnosis for the region of interest A1 of the subject more smoothly.

It is not necessary to store the two-dimensional ultrasound images U of the plurality of frames in the memory 43 of the server 3, and instead, one volume rendering image X is stored in the memory 43. Therefore, it is possible to suppress an increase in the amount of data to be stored in the server 3.

Next, in Step S9, the database management unit 42 of the server 3 associates the volume rendering image X with the two-dimensional ultrasound image U and the radiation image linked to the tag of the volume rendering image X, on the basis of the reference information stored in the tag of the volume rendering image X transmitted to the server-side communication unit 41 via the network NW, and creates a database from the relationship between these pieces of image data. The image data of which the relationship is databased is stored in the memory 43 of the server 3.

Subsequently, in Step S10, for example, in a case where a command to browse the two-dimensional ultrasound image U stored in the server 3 is input by the input operation of the user such as a doctor via the input device 55 of the viewer 4, information requesting the transmission of the two-dimensional ultrasound image U is transmitted from the viewer 4 to the server 3, the two-dimensional ultrasound image U stored in the memory 43 of the server 3 in Step S9 is transmitted from the server-side communication unit 41 to the viewer 4, and the two-dimensional ultrasound image U received by the viewer-side communication unit 51 is displayed on the viewer-side monitor 53.

The two-dimensional ultrasound image U displayed on the viewer-side monitor 53 includes the region of interest A1 as illustrated in Fig. 4.

In subsequent Step S11, the viewer control unit 54 determines whether or not the region of interest A1 in the two-dimensional ultrasound image U displayed on the viewer-side monitor 53 is designated by the user. In a case where it is determined that the region of interest A1 is not designated by the user, the processing returns to Step S11. On the other hand, in a case where it is determined that the region of interest A1 is designated by the user, the processing proceeds to Step S12.

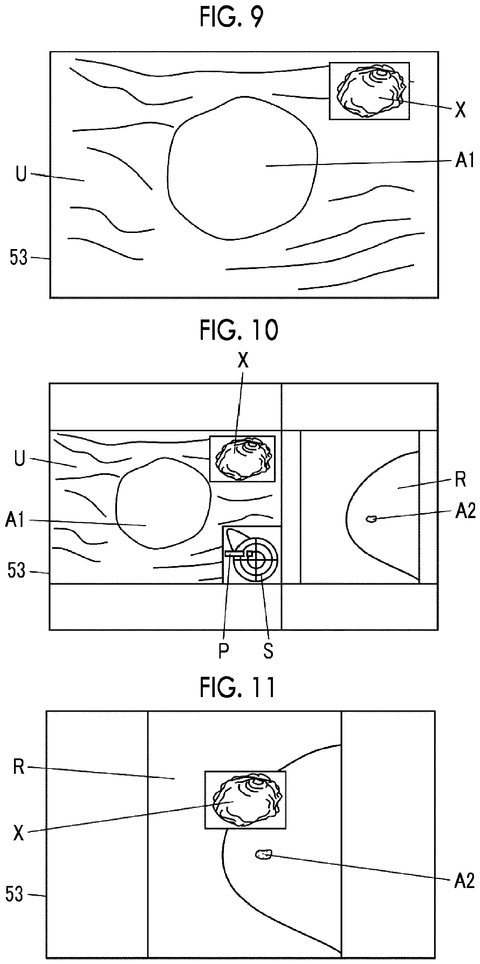

In Step S12, the volume rendering image X associated with the two-dimensional ultrasound image U displayed on the viewer-side monitor 53 is displayed near the two-dimensional ultrasound image U, as illustrated in Fig. 9.

In this case, a command to transmit the volume rendering image X associated with the two-dimensional ultrasound image U displayed on the viewer-side monitor 53 is transmitted from the viewer control unit 54 to the server 3 via the viewer-side communication unit 51. The server control unit 44 reads out the volume rendering image X linked to the two-dimensional ultrasound image U displayed on the viewer-side monitor 53, from the memory 43 on the basis of the command received from the viewer 4, and transmits the volume rendering image X from the server-side communication unit 41 to the viewer 4. The volume rendering image X is received by the viewer-side communication unit 51, is sent to the display control unit 52, and is displayed on the viewer-side monitor 53.

Thereby, the user can easily understand the three-dimensional shape of a location suspected to be a lesion part of the subject indicated by the region of interest A1 on the two-dimensional ultrasound image U.

In a case where Step S12 is completed, the operation of the ultrasound system 1 according to the flowchart of Fig. 8 is ended.

As described above, with the ultrasound system 1 according to the embodiment of the present invention, since the volume rendering image X of the region of interest A1 is generated on the basis of the two-dimensional ultrasound images U of the plurality of frames, it is possible to allow the user to easily understand the three-dimensional shape of the region of interest A1 suspected to be a lesion part in the subject, and to improve the diagnostic accuracy for the region of interest A1.

Further, the volume rendering image X generated in the diagnostic apparatus main body 6 is linked to the reference information regarding the region of interest A1, and the volume rendering image X linked to the reference information is transmitted to the server 3, and is stored in the memory 43 of the server 3. Therefore, for example, as compared with a case where the two-dimensional ultrasound images U of the plurality of frames are transmitted to the server 3 and the volume rendering image X is generated in the server 3, the server 3 can acquire the volume rendering image X more quickly, and the user can use the server 3 to perform a diagnosis for the region of interest A1 of the subject more smoothly.

It is not necessary to store the two-dimensional ultrasound images U of the plurality of frames in the memory 43, and instead, one volume rendering image X is stored in the memory 43. Therefore, it is possible to suppress an increase in the amount of data to be stored in the server 3.

In the ultrasound system 1, the ultrasound image generation unit 22 is provided in the diagnostic apparatus main body 6, but may be provided in the ultrasound probe 5 instead of being provided in the diagnostic apparatus main body 6.

It has been described that the ultrasound probe 5 and the diagnostic apparatus main body 6 are connected to each other by wired communication, but the ultrasound probe 5 and the diagnostic apparatus main body 6 can be connected to each other by wireless communication.

The diagnostic apparatus main body 6 includes one memory 25, but can include a plurality of memories depending on the application, for example.

Similarly, the server 3 can include a plurality of memories instead of one memory 43.

It has been described that the server 3 and the viewer 4 are independently present, and are connected to each other via the network NW, but the server 3 and the viewer 4 can also be integrally configured.

Further, in the description regarding the operation of the ultrasound system 1 using the flowchart of Fig. 8, an example has been described in which the ultrasound system 1 is constructed in a hospital, but any one of the ultrasound diagnostic apparatus 2, the server 3, or the viewer 4 may be installed in a remote facility such as another hospital.

It has been described that the radiation image stored in the server 3 is transmitted to the diagnostic apparatus main body 6 in a case where an examination for the subject is started in Step S1, but the radiation image can be transmitted from a radiological diagnostic apparatus (not illustrated) to the diagnostic apparatus main body 6, and the radiation image can be stored in the memory 25.

It has been described that, in Step S2, the ultrasound probe 5 is moved in a state of being in contact with the body surface of the subject by the user, but in a case where the region of interest A1 as an examination target is sufficiently small, the two-dimensional ultrasound images U of the plurality of frames in which the region of interest A1 is shown can be captured by so-called electronic scanning or the like while the position of the ultrasound probe 5 is fixed. In this manner, in a case where the examination for the subject can be completed with the position of the ultrasound probe 5 being fixed, the positional information of the ultrasound probe 5 may not be used, and thus, the ultrasound probe 5 may not include the position sensor 13.

The two-dimensional ultrasound image U is generated in Step S2, and the positional information of the ultrasound probe 5 is acquired in Step S3. However, Step S2 may be performed after Step S3, or Step S2 and Step S3 may be simultaneously performed.

It has been described that, in Step S6, the VR image generation unit 26 automatically extracts the region of interest A1 from each of the two-dimensional ultrasound images U of the plurality of frames, but the method of designating the region of interest A1 is not limited thereto. For example, the region of interest A1 can be designated on the basis of the user's input operation via the input device 30.

In Step S6, a plurality of regions of interest A1 may be extracted from one ultrasound image U in some cases. In this case, for example, in Step S6, using the extraction of a plurality of regions of interest A1 from any one of the two-dimensional ultrasound images U of the plurality of frames as a trigger, the two-dimensional ultrasound image U including the plurality of regions of interest A1 is displayed on the main body-side monitor 24, and one of the plurality of regions of interest A1 is selected by the user via the input device 30 of the diagnostic apparatus main body 6. Further, the information on the region of interest A1 selected by the user is sent to the VR image generation unit 26 via the main body control unit 29, and the region of interest A1 that is the same as the region of interest A1 selected by the user is extracted from the two-dimensional ultrasound images U of the plurality of frames by the VR image generation unit 26.

In subsequent Step S7, the volume rendering image X of the region of interest A1 selected by the user is generated on the viewer 4 side, and the reference information is stored in the tag of the volume rendering image X in Step S8. In the reference information, information on coordinates of the region of interest A1, which is selected by the user, in the two-dimensional ultrasound image U is included. The relationship between the reference information including the information on the coordinates of the region of interest A1 selected by the user and the volume rendering image X is databased in Step S9, and is stored in the memory 43 of the server 3.

In this state, the two-dimensional ultrasound image U is displayed on the viewer-side monitor 53 in Step S10, and in a case where the region of interest A1 that is the same as the region of interest A1 selected by the user in Step S6 is selected in Step S11, the volume rendering image X linked to the information on the coordinates of the region of interest A1 is displayed on the viewer-side monitor 53 by being superimposed on the two-dimensional ultrasound image U.

In a case where a radiation image of the breast of the subject is captured, usually, radiation images for a region of interest A2 suspected to be a lesion part are often captured in two different directions of so-called cranio-caudal (CC) direction and mediolateral-oblique (MLO) direction. In this case, two radiation images captured from two different directions are stored in the memory 43 of the server 3. Further, the reference information linking unit 27 of the diagnostic apparatus main body 6 can store the identification information of two radiation images stored in the memory 43 of the server 3, as the reference information in the tag of the volume rendering image X.

In a case where the two-dimensional ultrasound image U of the breast of the subject is captured, usually, two tomographic planes orthogonal to each other for the region of interest A1 suspected to be a lesion part are imaged, and the two two-dimensional ultrasound images U are often referred to in a case of the diagnosis of the subject. For example, in Step S8, the VR image generation unit 26 can select the two-dimensional ultrasound image U of one representative frame among the two-dimensional ultrasound images U of the plurality of frames stored in the memory 25 of the diagnostic apparatus main body 6, generate the two-dimensional ultrasound image U representing a tomographic plane orthogonal to the tomographic plane represented by the two-dimensional ultrasound image U of the one representative frame on the basis of the two-dimensional ultrasound images U of the plurality of frames and the positional information of the ultrasound probe 5, and sent the two-dimensional ultrasound images U of the two frames to the reference information linking unit 27.

In this case, the reference information linking unit 27 can store the identification information of the two-dimensional ultrasound images U of the two frames representing the tomographic planes along two different directions, in the tag of the volume rendering image X. The two-dimensional ultrasound images U of the two frames are transmitted from the main body-side communication unit 21 to the server 3 via the network NW, are associated with the volume rendering image X by the database management unit 42, and then are stored in the memory 43 of the server 3.

It has been described that in a case where it is determined in Step S11 that the region of interest A1 on the two-dimensional ultrasound image U displayed on the viewer-side monitor 53 is designated by the user, in Step S12, the volume rendering image X is displayed near the region of interest A1 on the two-dimensional ultrasound image U as illustrated in Fig. 9. However, other information linked to the volume rendering image X can be displayed on the viewer-side monitor 53.

For example, as illustrated in Fig. 10, in addition to the volume rendering image X, a radiation image R linked to the volume rendering image X can be displayed on the viewer-side monitor 53. In the example illustrated in Fig. 10, the radiation image R includes the region of interest A2 corresponding to the region of interest A1 in the two-dimensional ultrasound image U.

For example, a so-called schema S and a probe mark P disposed on the schema S may be displayed on the viewer-side monitor 53 on the basis of the positional information of the ultrasound probe 5 in a case where the two-dimensional ultrasound image U linked to the volume rendering image X is captured. In the example illustrated in Fig. 10, the schema S schematically representing the breast of the subject is superimposed and displayed on the two-dimensional ultrasound image U.

As illustrated in Fig. 11, instead of the two-dimensional ultrasound image U, the radiation image R can be displayed on the viewer-side monitor 53, and using the selection of the region of interest A2 in the radiation image R by the user via the input device 55 of the viewer 4 as a trigger, the volume rendering image X linked to the radiation image R can be displayed on the viewer-side monitor 53 by being superimposed on the radiation image R.

In this case, the user operating the viewer 4 can easily understand the three-dimensional shape of the region of interest A2 in the radiation image R, and thus the diagnostic accuracy for the region of interest A2 can be improved.

### Explanation of References

1: ultrasound system
2: ultrasound diagnostic apparatus
3: server
4: viewer
5: ultrasound probe
6: diagnostic apparatus main body
11: transducer array
12: transmission and reception circuit
13: position sensor
16: pulser
17: amplification unit
18: AD conversion unit
19: beam former
21: main body-side communication unit
22: ultrasound image generation unit
23, 52: display control unit
24: main body-side monitor
25, 43: memory
26: VR image generation unit
27: reference information linking unit
29: main body control unit
30, 55: input device
31: main body-side processor
32: signal processing unit
33: DSC
34: image processing unit
41: server-side communication unit
42: database management unit
44: server control unit
51: viewer-side communication unit
53: viewer-side monitor
54: viewer control unit
A1, A2: region of interest
NW: network
P: probe mark
R: radiation image
S: schema
U: two-dimensional ultrasound image
X: volume rendering image

## Claims

1. An ultrasound system comprising:
an ultrasound probe;
an image generation unit that generates two-dimensional ultrasound images of a plurality of frames in which a region of interest of a breast of a subject is imaged by performing transmission and reception of an ultrasound beam with respect to the subject using the ultrasound probe;
a volume rendering image generation unit that generates a volume rendering image including the region of interest on the basis of the two-dimensional ultrasound images of the plurality of frames; and
a reference information linking unit that links the volume rendering image to reference information regarding the region of interest.

2. The ultrasound system according to claim 1,
wherein the reference information linking unit stores identification information of a radiation image in which the region of interest is imaged, as the reference information in a tag of the volume rendering image.

3. The ultrasound system according to claim 2,
wherein the radiation image includes two radiation images obtained by imaging the region of interest from two different directions, and
the reference information linking unit stores identification information of each of the two radiation images, as the reference information in the tag of the volume rendering image.

4. The ultrasound system according to claim 1,
wherein the reference information linking unit stores identification information of another two-dimensional ultrasound image in which the region of interest is imaged, as the reference information in a tag of the volume rendering image.

5. The ultrasound system according to claim 4,
wherein the other two-dimensional ultrasound image includes two two-dimensional ultrasound images obtained by imaging two tomographic planes orthogonal to each other for the region of interest, and
the reference information linking unit stores identification information of each of the two two-dimensional ultrasound images, as the reference information in the tag of the volume rendering image.

6. The ultrasound system according to any one of claims 1 to 5, further comprising:
an ultrasound diagnostic apparatus; and
a server connected to the ultrasound diagnostic apparatus,
wherein the ultrasound diagnostic apparatus includes the ultrasound probe, the image generation unit, the volume rendering image generation unit, the reference information linking unit, and a communication unit that transmits the volume rendering image linked to the reference information, to the server.

7. The ultrasound system according to claim 6, further comprising:
a viewer connected to the server,
wherein the viewer includes
an input device through which a user performs an input operation, and
a monitor that displays a radiation image or the two-dimensional ultrasound image in which the region of interest is imaged, and
in a case where the region of interest in the radiation image or the two-dimensional ultrasound image displayed on the monitor is designated by the user via the input device, the volume rendering image including the region of interest is displayed on the monitor.

8. The ultrasound system according to claim 7,
wherein in a case where the region of interest in the radiation image or the two-dimensional ultrasound image displayed on the monitor is designated by the user via the input device, the radiation image or the two-dimensional ultrasound image and the volume rendering image in each of which the region of interest is imaged are displayed on the monitor.

9. The ultrasound system according to any one of claims 1 to 8,
wherein the volume rendering image generation unit generates the volume rendering image using surface rendering.

10. The ultrasound system according to any one of claims 1 to 9, further comprising:
an image memory that stores the volume rendering image.

11. A control method of an ultrasound system, the control method comprising:
generating two-dimensional ultrasound images of a plurality of frames in which a region of interest of a breast of a subject is imaged by performing transmission and reception of an ultrasound beam with respect to the subject using an ultrasound probe;
generating a volume rendering image including the region of interest on the basis of the two-dimensional ultrasound images of the plurality of frames; and
linking the volume rendering image to reference information regarding the region of interest.
